# EUROPÄISCHE PATENTSCHRIFT

(11) **EP 0 590 428 B1**
(45) Veröffentlichungstag und Bekanntmachung des Hinweises auf die Patenterteilung: **15.12.1999**
(21) Anmeldenummer: 93114934.8
(22) Anmeldetag: 16.09.1993
(51) Int. Cl.: C07D 471/04, C07D 475/00, C07D 487/04, A61K 31/495

(54) **Azachinoxaline, Verfahren zu ihrer Herstellung und ihre Verwendung**
Azaquinoxalines, process for their preparation, and their use
Azaquinoxalines, procédé pour leur préparation et leur utilisation

(30) Priorität: 26.09.1992 DE 4232392
(43) Veröffentlichungstag der Anmeldung: 06.04.1994
(73) Patentinhaber: HOECHST AKTIENGESELLSCHAFT, 65929 Frankfurt am Main (DE)
(72) Erfinder: Billhardt-Troughton, Uta-Maria, Dr., Raleigh, N.C. 27606 (US); Rösner, Manfred, Dr., D-65817 Eppstein/Ts. (DE); Bender, Rudolf, Dr., D-65812 Bad Soden/Ts. (DE); Meichsner, Christoph, Dr., D-65835 Liederbach (DE)

(56) Entgegenhaltungen:
- EP-A- 0 010 426
- EP-A- 0 162 776
- EP-A- 0 311 378
- EP-A- 0 320 136
- EP-A- 0 509 398
- US-A- 4 077 955
- CHEMOTHERAPY, Bd. 21, 1975, BASEL, CH Seiten 221 - 230 M.A. VERINI ET AL. 'Antiviral ...'
- CHEMISCHE BERICHTE, Bd. 107, Nr. 3, 1974, Seiten 785 - 795 W. PFLEIDERER 'Pteridine ...'
- JOURNAL OF THE CHEMICAL SOCIETY, November 1963, LONDON, GB Seiten 5156 - 5166 A. ALBERT ET AL. 'Triazanaphthalenes ...'
- JOURNAL OF MEDICINAL CHEMISTRY, Bd. 16, Nr. 11, 1973, Seiten 1296 - 1298 H. BERNER ET AL. 'Antivirals ...'

## Beschreibung

Gegenstand der vorliegenden Erfindung sind Azachinoxaline, Verfahren zu ihrer Herstellung und ihre Verwendung.

Die Grundgerüste der Pteridine und Pyridopyrazine sind seit langem bekannt (D. J. Brown, Fused Pyrimidines Vol. III: Pteridines in The Chemistry of Heterocyclic Compounds, E. C. Taylor und A. Weissberger, Eds., John Wiley & Sons, Inc. 1988; G. W. H. Cheeseman, R. F. Cookson, Condensed Pyrazines in The Chemistry of Heterocyclic Compounds, E. C. Taylor und A. Weissberger, Eds., John Wiley & Sons, Inc. 1979). Über Pyrazinopyridazine findet man in der Literatur erst wenig (R. N. Castle, Condensed Pyridazines Including Chinolines and Phthalazines in The Chemistry of Heterocyclic Compounds, E. C. Taylor und A. Weissberger, Eds., John Wiley & Sons, Inc. 1973).

Die ungesättigten Derivate Xanthopterin und Isoxanthopterin gehören zu den wichtigsten natürlich vorkommenden Pteridinen. Ihre Antitumoraktivität stimulierte eine Reihe synthetischer Arbeiten (z. B. E. C. Taylor, R. F. Abdulla, K. Tanaka und P. A. Jacobi J. Org. Chem. 1975, 40, 2341; W. Pfleiderer Chem. Ber. 1974, 107, 785).

Tetrahydro-2-oxo-8-amino-pyrido[2,3-b]pyrazin-7-carbonsäuren werden in einer Patentanmeldung von Squibb & Sons, Inc. als antiinflammatorische Verbindungen und als Sedativa beschrieben (US 4077-955, 17. 2. 1977). In einer Patentanmeldung von Ferrosan A/S werden 3-substituierte 4,5-Dihydro-5-isopropyl-4-oxo-imidazo[1,5-a]chinoxaline und -6-azachinoxaline mit starker Affinität zum Benzodiazepinrezeptor (EP 320-136-A, 8. 12. 1987) beschrieben. N-Carboxymethyl-pyrido[2,3-b]pyrazin-2(1H)-one werden unter anderem in einer Patentanmeldung von Carpibem SA als Aldosereduktase Inhibitoren beansprucht (EP 162-776-A, 18. 5. 1984).

In der EP 0 509 398 wird die anitvirale Aktivität von Chinoxalinen beschrieben. Chemotherapy, Bd. 21, 1975, Seiten 221 - 230 erläutert die antivirale Wirksamkeit von Pyrazino-Pyridazin Derivaten. In der EP 0 010 426 wird die Verwendung von 3,4 dihydro-3-oxo-2-chinoxalinen, die an den Ringstickstoffatomen unsubstituiert sind, als anivirale Mittel beschrieben. Im Journal of Medicinal Chemistry, Bd. 16, Nr. 11, 1973, Seiten 1296 - 1298 wird über Untersuchungen hinsichtlich der Wirkung von 2-(α-Hydroxybenzyl)imidazo[4,5-c]pyridine gegen bestimmte Polioviren berichtet. In der EP 0 311 378 werden 6-substituierte Alkoxy-2-oxo-1,2-dihydrochinoxalin Derivate und ihre Wirksamkeit als gerinnungshemmende Mittel beschrieben.

Es wurde nun überaschenderweise gefunden, daß bestimmte Azachinoxaline eine antivirale Wirksamkeit aufweisen. Erfindungsgegenstand sind demzufolge 1) Verbindungen der Formel I, 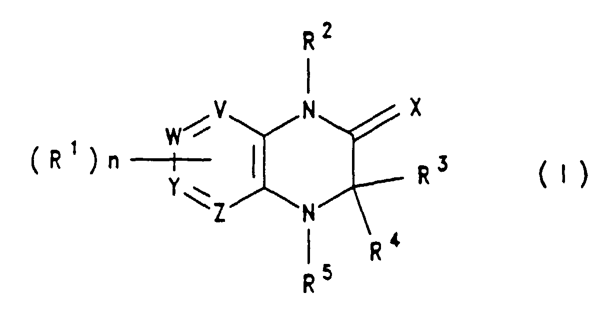 sowie deren tautomere Form der Formel la, 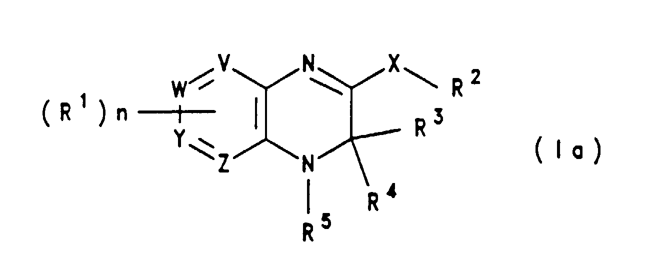 worin bedeuten
n null,
oder eins,
R¹
Fluor, Chlor, Trifluormethyl, C₁-C₃-Alkyl,
V, W und Y bedeuten CH oder CR¹ und Z bedeutet N,
X bedeutet Sauerstoff oder Schwefel,
R² und R⁵ können gleich oder verschieden, unabhängig voneinander
Wasserstoff, Hydroxy,
C₁-C₃-Alkyl, C₂-C₆-Alkenyl, C₁-C₄-Alkyloxycarbonyl, C₂-C₄-Alkenyloxycarbonyl,
oder einen 2-, 3- oder 4-Picolylrest bedeuten,
R³ bedeutet Wasserstoff und R⁴
C₁-C₄-Alkyl, gegebenenfalls substituiert mit C₁-C₂-Alkylthio, C₁-C₂-Alkylsulfonyl oder C₁-C₂-Alkylsulfinyl;
mit Ausnahme der Verbindungen, in denen R² und R⁵ Wasserstoff bedeuten.

Die in den vorangegangenen Definitionen genannten Alkylgruppen können geradkettig oder verzweigt sein. Beispiele sind die Methyl-, Ethyl-, Propyl-, 1-Methylethyl-, gruppe.

Die in den vorangegangenen Definitionen genannten Alkenylgruppen können geradkettig oder verzweigt sein und enthalten 1 bis 3 Doppelbindungen. Beispiele sind die 2-Propenyl-, 1-Methylethenyl-, 2-Butenyl-, 3-Butenyl-, 2-Methyl-2-propenyl-, 3-Methyl-2-butenyl-, 2,3-Dimethyl-2-butenyl, und Pentadienylgruppe und ähnliche.

Die obengenannten Substituenten R⁴ sind vorzugsweise 3-fach, besonders bevorzugt 2-fach, insbesondere einfach mit den jeweils angegebenen Substituenten substituiert.

In Abhängigkeit von den verschiedenen Substituenten können Verbindungen der Formeln I und la mehrere asymmetrische Kohlenstoffatome besitzen. Gegenstand der Erfindung sind deshalb sowohl die reinen Stereoisomeren als auch Mischungen derselben, wie z. B. das zugehörige Racemat. Die reinen Stereoisomeren der Verbindungen der Formeln I und la lassen sich durch bekannte Methoden oder in Analogie zu bekannten Methoden direkt herstellen oder nachträglich trennen.

Zum Gegenstand der vorliegenden Erfindung gehört weiterhin ein Verfahren zur Herstellung von Verbindungen der Formel I und la wie oben unter 1) erläutert, dadurch gekennzeichnet, daß A) zur Herstellung von Verbindungen der Formel I mit X gleich Sauerstoff und den Resten R¹, R², R³, R⁴, R⁵ und n wie unter 1) definiert, eine Verbindung der Formel II, 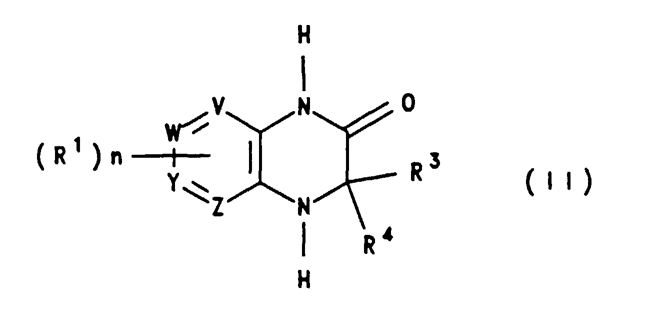 wobei für R¹, R³ und R⁴ die unter 1) genannten Definitionen gelten, mit einer Verbindung der Formel III,

R-L¹ (III)

wobei R die oben unter 1) genannten Bedeutungen für R⁵ und R² mit Ausnahme von Wasserstoff, Hydroxy, Acylamino hat und L¹ eine Abgangsgruppe ist, umsetzt
oder daß
B) Verbindungen der Formel I, mit X gleich Schwefel und den Resten R¹, R², R³, R⁴ und R⁵ wie unter 1) definiert, hergestellt werden durch Reaktion einer Verbindung der Formel I, wobei X Sauerstoff ist und für R¹ bis R⁵ die unter 1) beschriebenen Definitionen gelten, mit einem Schwefelungsreagenz,
oder daß
C) Verbindungen der Formel la, wobei X und die Reste R¹ bis R⁵ wie unter 1) definiert, hergestellt werden, indem man eine Verbindung der Formel IV, 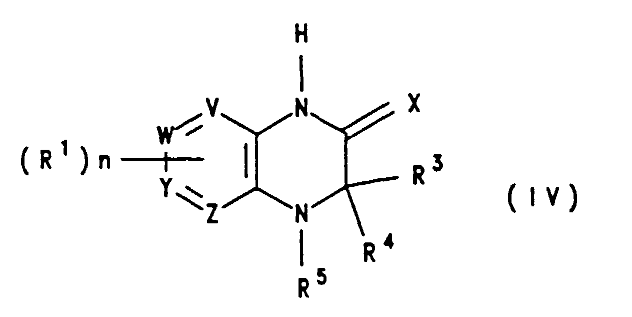 bzw. lVa, 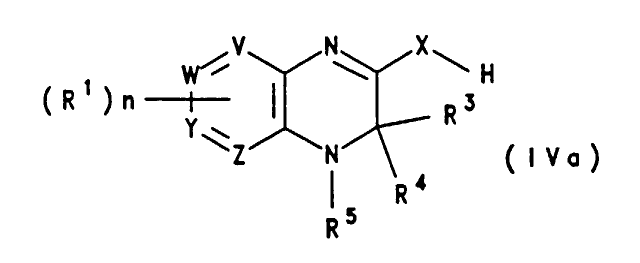 wobei für X, R¹, R³, R⁴ und R⁵ die unter 1) genannten Definitionen gelten, mit einer Verbindung der Formel III,

R-L¹ (III)

wobei R die oben unter 1) genannten Bedeutungen für R² mit Ausnahme von Wasserstoff, Hydroxy, hat und L¹ eine Abgangsgruppe ist, umsetzt,
oder daß
D) Verbindungen der Formel I mit X gleich Sauerstoff und den Resten R¹ bis R⁵ wie unter 1) definiert durch Cyclisierung einer Verbindung der Formel V, 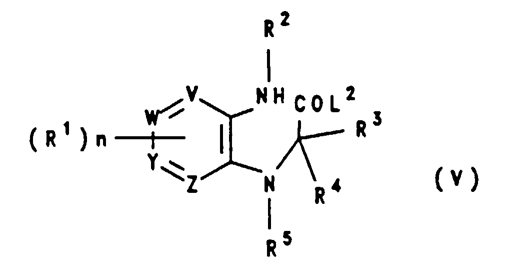 mit R¹ bis R⁵ wie unter 1) definiert und L² gleich Hydroxy, Alkoxy, ggf. halogeniertes Acyloxy, Chlor, Brom oder Jod, hergestellt werden,
oder daß
F) Verbindungen der Formel I, wobei X gleich Sauerstoff und R¹ bis R⁵ wie unter 1) definiert sind, hergestellt werden aus Verbindungen der Formel VI, 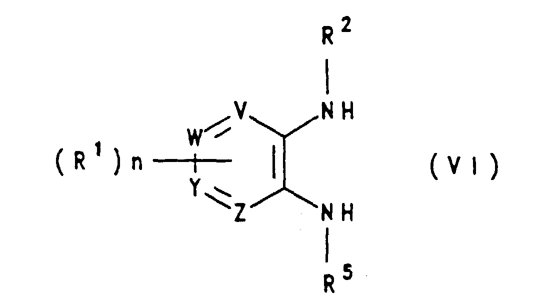 mit R¹, R² und R⁵ wie unter 1) definiert, durch Umsetzung mit Chloroform oder Bromoform und einer Carbonylverbindung der Formel XIII,

R³-CO-R⁴ (XIII)

mit R³ und R⁴ wie unter 1) definiert oder mit α-(Trihalogenmethyl)-alkanolen der Formel XIV,

Hal₃C-C(OH)-R³R⁴ (XIV),

worin Hal für Cl, Br oder J steht und in denen R³ und R⁴ wie unter 1) definiert sind,
oder daß
G) Verbindungen der Formel I, mit X gleich Sauerstoff, R¹, R², R³ und R⁴ wie unter 1) definiert und R⁵ C₁-C₃-Alkyl; C₂-C₆-Alkenyl; durch reduktive Alkylierung einer Verbindung der Formel I, wobei R⁵ Wasserstoff und X Sauerstoff sind und für R¹, R², R³ und R⁴ die unter 1) genannten Definitionen gelten, mit einer Carbonylverbindung der Formel XV,

R"-C(=O)-R"' (XV)

wobei R" und R"' gleich oder verschieden, unabhängig voneinander, C₁-C₃-Alkyl, C₂-C₆-Alkenyl, hergestellt werden.

Die obengenannte Methode A) läuft vorzugsweise unter folgenden Bedingungen ab:

Der Substituent L¹ in der Formel III ist eine geeignete Abgangsgruppe, wie z. B. Chlor, Brom oder Jod, ein geeignetes Radikal der Schwefelsäure, ein aliphatischer oder aromatischer Sulfonsäureester oder ggf. halogeniertes Acyloxy.

Die Umsetzung wird zweckmäßigerweise in einem inerten Lösungsmittel durchgeführt. Geeignet sind z. B. aromatische Kohlenwasserstoffe wie Toluol oder Xylol, niedere Alkohole wie Methanol, Ethanol oder 1-Butanol, Ether wie Tetrahydrofuran oder Glycoldimethylether, dipolar aprotische Lösungsmittel wie N,N-Dimethylformamid, N-Methyl-2-pyrrolidon, Acetonitril, Nitrobenzol, Dimethylsulfoxid oder Gemische dieser Lösungsmittel.

Auch Zweiphasensysteme mit wäßrigen Lösungen von Basen in Gegenwart eines Phasentransferkatalysators, wie z. B. Benzyltriethylammoniumchlorid, sind möglich.

Die Anwesenheit einer geeigneten Base z. B. eines Alkali- oder Erdalkalimetallcarbonats oder -hydrogencarbonats wie Natriumcarbonat, Calciumcarbonat oder Natriumbicarbonat, eines Alkali- oder Erdalkalihydroxids wie Kaliumhydroxid oder Bariumhydroxid, eines Alkoholats wie Natriumethanolat oder Kalium-tert.-butylat, einer lithiumorganischen Verbindung wie Butyllithium oder Lithiumdiisopropylamid, eines Alkali- oder Erdalkalihydrids wie Natriumhydrid oder Calciumhydrid, ein Alkalifluorid wie Kaliumfluorid oder einer organischen Base wie Triethylamin oder Pyridin zum Auffangen der bei der Reaktion freiwerdenden Säure kann nützlich sein.
In manchen Fällen ist der Zusatz eines Jodsalzes, z. B. Kaliumjodid, angebracht. Die Reaktion wird gewöhnlich bei Temperaturen zwischen - 10 und 160 °C durchgeführt, vorzugsweise bei Raumtemperatur.

Für diese Umsetzung müssen etwaige nucleophile Substituenten wie z. B. Hydroxy-, Mercapto-, oder Aminogruppen mit Ausnahme der 1- und/oder 4-Position in Verbindungen der Formel II oder in III, vor Durchführung der Reaktion in geeigneter Weise derivatisiert oder mit wieder abspaltbaren gebräuchlichen Schutzgruppen wie z. B. Acetyl oder Benzyl versehen werden.

Für die Umsetzung wie zuvor unter B) beschrieben wird vorzugsweise als Schwefelungsreagenz 2,4-Bis(4-methoxyphenyl)-1,3-dithia-2,4-diphosphetan-2,4-disulfid (Lawessons Reagenz), Bis(tricyclohexylzinn)sulfid, Bis(tri-n-butylzinn)sulfid, Bis(triphenylzinn)sulfid, Bis(trimethylsilyl)sulfid oder Phosphorpentasulfid verwendet.

Die Reaktion wird zweckmäßigerweise in einem inerten organischen Lösungsmittel, wie z. B. Schwefelkohlenstoff, Toluol oder Xylol, bei Raumtemperatur oder höher, bevorzugt bei der Siedetemperatur des Reaktionsgemisches und möglichst unter wasserfreien Bedingungen durchgeführt. Bei Verwendung der erwähnten Zinn- oder Silylsulfide ist es angebracht, die Schwefelungsreaktion in Gegenwart einer Lewissäure wie Bortrichlorid durchzuführen.

In Gegenwart anderer Carbonylgruppen in einer Verbindung der Formel I, z. B. in einer Verbindung, wo X gleich Sauerstoff und einer oder mehrere Reste R¹ bis R⁶ gleich Acyl sind, ist das Carbonyl vor der Schwefelungsreaktion nach bekannten Methoden durch eine geeignete Schutzgruppe, z. B. durch Acetalisierung, zu schützen; anschließende Schutzgruppenabspaltung führt zur gewünschten Verbindung.

Für die oben unter C) beschriebene Umsetzung ist L¹ eine geeignete Abgangsgruppe, vorzugsweise Chlor, Brom oder Jod, ein geeignetes Radikal der Schwefelsäure, ein aliphatischer oder aromatischer Sulfonsäureester oder ggf. halogeniertes Acyloxy.

Die Reaktionsbedingungen für diese Umsetzung entsprechen denjenigen der Methode A.

Die unter D) beschriebene Cyclisierung findet in einem geeigneten Lösungsmittel statt wie z. B. Methanol, Ethanol, N,N-Dimethylformamid oder N-Methylpyrrolidon in Gegenwart einer Base; geeignet sind Alkali- oder Erdalkalimetallcarbonate oder -hydrogencarbonate wie Natriumcarbonat, Calciumcarbonat oder Natriumbicarbonat, Alkali- oder Erdalkalihydroxide wie Kaliumhydroxid oder Bariumhydroxid, Alkoholate wie Natriumethanolat oder Kalium-tert.-butylat, lithiumorganische Verbindungen wie Butyllithium oder Lithiumdiisopropylamid, Alkali- oder Erdalkalihydride wie Natriumhydrid oder Calciumhydrid oder eine organische Base wie Triethylamin oder Pyridin - letztere können auch als Lösungsmittel verwendet werden, oder organische oder anorganische Säuren wie Eisessig, Trifluoressigsäure, Salzsäure oder Phosphorsäure. Die Reaktion wird vorzugsweise bei Temperaturen zwischen 20 und 120 °C, besonders bevorzugt bei Raumtemperatur durchgeführt.

Die Verbindungen der Formel V, wobei R¹ bis R⁵ und L² wie unter 1) bzw. D) definiert sind, können aus Verbindungen der Formel VI, 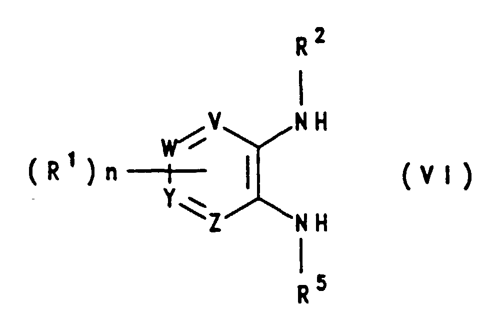 wobei R¹, R² und R⁵ wie unter 1) definiert sind, durch Alkylierung mit einer Verbindung der Formel VII, 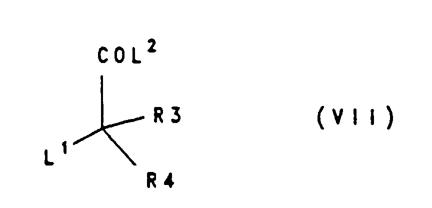 wobei R³, R⁴ und L² wie unter 1) bzw. D) und L¹ wie unter A) definiert sind, erhalten werden. Die Reaktionsbedingungen für diese Alkylierung entsprechen den zur Methode A) gegebenen.

Unter geeigneten Bedinungen findet dabei gleichzeitig der Ringschluß zum Azadihydrochinoxalin der Formel I statt.

Verbindungen der Formel V, in denen R¹, R³ bis R⁵ und L² wie unter 1) bzw. D) definiert sind und R² Wasserstoff bedeutet, können auch aus Verbindungen der Formel VIII, 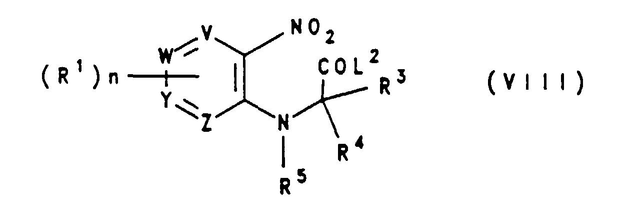 mit R¹, R³ bis R⁵ und Y wie unter 1) bzw. D) definiert, hergestellt werden, indem man die Nitrogruppe nach bekannten Verfahren zur Aminogruppe reduziert.

Unter geeigneten Bedingungen, z. B. bei Reduktion in Anwesenheit von Säure, findet dabei gleichzeitig der Ringschluß zum Azadihydrochinoxalin der Formel I statt.

Die Reduktion wird nach Standardmethoden (s. z. B. Methoden der organischen Chemie (Houben-Weyl), E. Müller (Herausgeber); G. Thieme Verlag, Stuttgart, 1957; Bd. XI/1, S. 360 - 490) z. B. mit Zinn(II)chlorid in Eisessig, TiCl₃ in Salzsäure, oder durch katalytische Hydrierung durchgeführt, wobei die Wahl des Reagenzes durch die chemische Stabilität der verschiedenen Substituenten R¹, R³ bis R⁵ bestimmt wird; ist z. B. einer der Reste Alkenyl, wird man die erste Methode wählen, um die Doppelbindung zu erhalten.
Die als Ausgangsmaterialien für die beschriebenen Synthesen benötigten ortho-Diaminopyridine, -pyridazine und -pyrimidine sind literaturbekannt oder käuflich oder können nach literaturbekannten Methoden synthetisiert werden.

N-ortho-Nitropyridyl-, N-ortho-Nitropyridazyl- und N-ortho-Nitropyrimidyl-aminosäurederivate der Formel VIII, wobei R¹ und R³ bis R⁵ wie unter 1) definiert sind und L² gleich OR⁷ ist, mit R⁷ gleich Wasserstoff, Alkyl, ggf. jeweils z. B. Halogen-substituiertes Phenyl, Benzyl oder 9-Fluorenylmethyl, bedeutet, können z. B. durch Aminierung von ortho-Halogennitropyridinen bzw. -pyrimidinen der Formel IX, 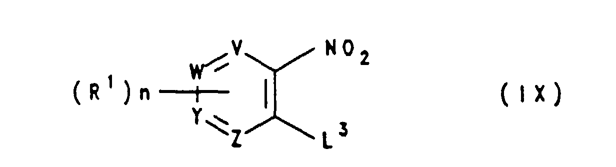 wobei R¹ wie unter 1) definiert ist und L³ Fluor, Chlor, Brom oder Jod bedeutet, mit Aminosäuren oder ihren Estern der Formel X, 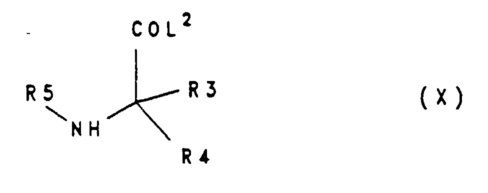 wobei R³, R⁴, R⁵ und L² wie unter 1) bzw. oben definiert sind, erhalten werden.

Die Reaktion kann in Gegenwart einer anorganischen oder organischen Hilfsbase, wie z. B. Natrium- oder Kaliumcarbonat, Natriumhydroxid oder Triethylamin durchgeführt werden. Günstig ist die Verwendung eines inerten Lösungsmittels bei Temperaturen zwischen 0 und 150 °C, vorzugsweise bei Rückflußtemperatur. Geeignete Lösungsmittel sind offenkettige oder cyclische Ether, z. B. Tetrahydrofuran oder Glycoldimethylether, aromatische Kohlenwasserstoffe, z. B. Toluol oder Chlorbenzol, Alkohole, z. B. Ethanol, Isopropanol oder Glycolmonomethylether, dipolar aprotische Lösungsmittel, z. B. N, N-Dimethylformamid, N-Methylpyrrolidon oder 1,3-Dimethyl-tetrahydro-2(1H)-pyrimidon.

Die N-ortho-Nitrophenylaminosäuren der Formel VIII mit L² gleich Hydroxy lassen sich falls gewünscht oder erforderlich nach wohlbekannten Standardmethoden in die Säurederivate der Formel VIII mit L² gleich Alkoxy, ggf. halogeniertes Acyloxy, Chlor, Brom oder Jod umwandeln.

Ortho-Halogennitropyridine und -Pyrimidine der Formel IX und Aminosäuren der Formel X sind literaturbekannt und käuflich oder lassen sich nach literaturbekannten Methoden herstellen.

Die oben unter F) beschriebene Umsetzung wird zweckmäßigerweise in einem Zweiphasensystem, bestehend aus einem organischen, nicht wassermischbaren Lösungsmittel oder Lösungsmittelgemisch, bestehend z. B. aus halogenierten Kohlenwasserstoffen, z. B. Dichlormethan oder 1,2-Dichlorethan oder aromatischen Kohlenwasserstoffen, z. B. Toluol oder Xylol und einer konzentrierten wäßrigen Lösung eines Alkali- oder Erdalkalimetallhydroxids, z. B. Natrium- oder Bariumhydroxid durchgeführt. Vorteilhaft ist die Anwesenheit eines Phasentransferkatalysators, wie z. B. Benzyltriethylammoniumchlorid oder Tetrabutylammoniumbromid.

Die Reaktion wird gewöhnlich bei Temperaturen zwischen 0 und 50 °C durchgeführt, vorzugsweise bei Raumtemperatur.

Substituenten in Verbindungen der Formeln VI und XIII, bzw. XIV, die unter den Reaktionsbedingungen nicht stabil sind, müssen durch solche ersetzt werden, die zu der benötigten Gruppe derivatisiert werden können. Die Substituenten können auch mit einer gebräuchlichen Schutzgruppe versehen sein, die nach der oben beschriebenen Umsetzung wieder entfernt werden kann.

Die unter G) beschriebene Reaktion findet vorzugsweise durch katalytische Hydrierung (mit Wasserstoff) in Gegenwart eines Hydrierkatalysators, z. B. Palladium auf Kohle, bei einem Wasserstoffdruck von 1 bis 5 bar, oder mittels eines Reduktionsmittels aus der Klasse der komplexen Metallhydride, wie Natriumborhydrid, Natriumtriacetoxyborhydrid oder Natriumcyanborhydrid statt. Zweckmäßigerweise wird die Umsetzung in einem inerten Lösungsmittel, wie niederen Alkoholen, z. B. Methanol oder Isopropanol, Ethern, z. B. Tetrahydrofuran oder Glycoldimethylether, halogenierten Kohenwasserstoffen, z. B. Dichlormethan oder 1,2-Dichlorethan, bei Temperaturen zwischen - 20 und 100 °C, vorzugsweise bei Raumtemperatur durchgeführt. Die Anwesenheit einer Säure, wie z. B. Essigsäure oder Trifluoressigsäure, oder einer Lewissäure, wie z. B. Titantetrachlorid, ist vorteihaft. In Gegenwart von Substituenten in Verbindungen der Formeln I und XV, welche unter den beschriebenen Bedingungen hydriert bzw. reduziert werden können, z. B. Oxo, ist die Verwendung eines Intermediates der Formeln I und XV, mit Substituenten, die nicht angegriffen werden, welche aber zu der benötigten Gruppe derivatisiert werden können, z. B. Hydroxy, nötig. Säurelabile Gruppen, wie z. B. Acetale oder unter den Reaktionsbedingungen reagierende Gruppen, wie z. B. primäre Amine, sind ebenfalls zu vermeiden bzw. mit einer grbräuchlichen Schutzgruppe zu versehen.

Weiterhin gehören zu dem Gegenstand der vorliegenden Erfindung Arzneimittel mit einem Gehalt an mindestens einer erfindungsgemäßen Verbindung. Darüber hinaus gehört zum Gegenstand der vorliegenden Erfindung die Verwendung von Verbindungen der Formel I, 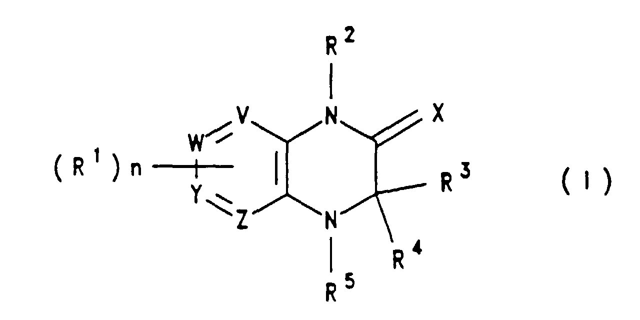 sowie deren tautomere Form der Formel la, 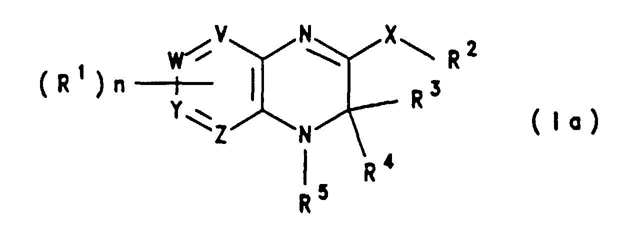 in denen n und die Substituenten R¹ bis R⁵ die unter 1) genannten Bedeutungen haben zur Herstellung von Arzneimitteln zur Behandlung von Viruserkrankungen insbesondere zur Behandlung von Krankheiten, hervorgerufen durch das Humane Immundefizienz Virus (HIV).

Die erfindungsgemäßen Arzneimittel können enteral (oral), parenteral (intravenös), rektal, subcutan, intramuskulär oder lokal (topisch) angewendet werden.

Sie können in Form von Lösungen, Pulvern, Tabletten (Kapseln einschließlich Microkapseln), Salben (Cremes oder Gele) oder Suppositorien verabreicht werden. Als Hilfsstoffe für derartige Formulierungen kommen die pharmazeutisch üblichen flüssigen oder festen Füllstoffe und Streckmittel, Lösemittel, Emulgatoren, Gleitstoffe, Geschmackskorrigentien, Farbstoffe und/oder Puffersubstanzen in Frage.

Als zweckmäßige Dosierung werden 0,1 - 30, vorzugsweise 0,2 - 10 mg/kg Körpergewicht ein oder mehrmals täglich verabreicht. Die verwendeten Dosierungseinheiten richten sich zweckmäßigerweise nach der jeweiligen Pharmakokinetik der verwendeten Substanz bzw. der verwendeten galenischen Zubereitung.

Die verwendete Dosierungseinheit der erfindungsgemäßen Verbindungen beträgt z. B. 1 - 1500 mg, vorzugsweise 50 - 500 mg.

Die erfindungsgemäßen Verbindungen können auch in Kombination mit anderen antiviralen Mitteln, wie z. B. Nucleosidanaloga, Proteaseinhibitoren oder Adsorptionsinhibitoren und Immunstimulantien, Interferonen, Interleukinen und Koloniestimulierenden Faktoren (z. B. GM-CSF, G-CSF, M-CSF) verabreicht werden.

Wirksamkeitstests
Prüfung von Präparaten gegen HIV in der Zellkultur Methodenbeschreibung

Medium:
RPMI pH 6,8
Komplettes Medium enthält zusätzlich 20 % foetales Kälberserum und 40 lU/ml rekombinantes Interleukin 2.

Zellen:
Aus frischem Spenderblut mittels ®Ficoll Gradienten-Zentrifugation isolierte Lymphozyten werden unter Zusatz von 2 µg/ml Phytohämagglutinin (Wellcome) in komplettem Medium 36 h bei 37 °C unter 5 % CO₂ kultiviert. Die Zellen werden nach Zusatz von 10 % DMSO bei einer Zelldichte von 5 x 10⁶ eingefroren und in flüssigem Stickstoff gelagert. Für den Versuch werden die Zellen aufgetaut, im RPMI-Medium gewaschen und im kompletten Medium 3 - 4 Tage kultiviert.

Ansatz:
Die Prüfpräparate wurden in DMSO gelöst und mit komplettem Medium auf eine Konzentration von 1 mg/ml eingestellt. In 24er Multiwell-Schalen wurden 0,4 ml Medium vorgelegt. Nach Zugabe von 0,1 ml des gelösten Präparates in die obere Reihe der Schale wurde durch Übertragung von jeweils 0,1 ml eine geometrische Verdünnungsreihe erzeugt. Präparatfreie Kontrollen enthielten stets 0,4 ml komplettes Medium mit 0,5 % DMSO. Lymphozytenkulturen mit einer Zellzahl von 5 x 10⁵ Zellen/ml wurden durch Zugabe 1/50 Volumen Überstand aus HIV-infizierten Lymphozytenkulturen infiziert. Der Titer dieser Kulturüberstände wurde durch Endpunktverdünnung mit 1 - 5 x 10⁶ infektiöse Einheiten/ml bestimmt. Nach 30 min Inkubation bei 37 °C wurden die infizierten Lymphozyten abzentrifugiert und im gleichen Volumen Medium wieder aufgenommen. Von dieser Zellsuspension wurden jeweils 0,6 ml in alle Vertiefungen der Testplatte gegeben. Die Ansätze wurden 3 Tage bei 37 °C inkubiert.

Auswertung:
Die infizierten Zellkulturen wurden unter dem Mikroskop auf Anwesenheit von Riesenzellen untersucht, die eine aktive Virusvermehrung in der Kultur anzeigen. Die geringste Präparatekonzentration, bei der keine Riesenzellen auftraten, wurde als Hemmkonzentration gegen HIV bestimmt. Zur Kontrolle wurden die Überstände aus den Kulturplatten mit Hilfe eines HIV-Antigentests entsprechend den Angaben des Herstellers (Organon) auf Anwesenheit von HIV-Antigen bestimmt.

Ergebnisse:
Die Ergebnisse dieses Tests zeigt Tabelle 1.

**Tabelle 1**

| Verbindung des Beispiels Nr. | T-Zellkulturassay MHK (µg/ml) |
|---|---|
| 2 | > 0.8 |
| 8 | 0.08 |
| 10 | 1.0 |
| 11 | 0.2 |
| 37 | > 1,0 |
| 41 | > 2,0 |

Untersuchung der Substanzen auf Hemmung der HIV-"Reverse Transkriptase"

Die Aktivität der Reversen Transkriptase (RT) wurde mit Hilfe eines "Scintillation Proximity Assay" (SPA) bestimmt. Das Reagenzkit für den RT-SPA wurde von Amersham/Buchler (Braunschweig) bezogen. Das Enzym RT (aus HIV in E. coli cloniert) stammte von der Firma HT-Biotechnology LTD, Cambridge, UK.

Ansatz:
Der Test wurde nach dem Methoden-Manual des Herstellers Amersham durchgeführt - mit folgenden Modifikationen:
- Dem "Assay"-Puffer wurde Rinderserumalbumin zu der Endkonzentration 0,5 mg/ml zugesetzt.
- Der Test wurde in Eppendorf-Reaktionsgefäßen mit 100 µl Ansatzvolumen durchgeführt.
- Das RT-Konzentrat des Herstellers (5000 U/ml) wurde in Tris-HCI Puffer 20 mM; pH 7,2; 30 % Glycerin auf eine Aktivität von 15 U/ml verdünnt.
- Die Inkubationszeit für die Ansätze betrug 60 min (37 °C).
- Nach Abstoppen der Reaktion und "Entwicklung" mit der Perlen-Suspension wurden 130 ml Ansatz in 4,5 ml Tris-HCI Puffer, 10 mM; pH 7,4; 0,15 M NaCI transferiert und die Tritium-Aktivität in einem β-Counter gemessen.

Substanzprüfung:
Für eine Vorprüfung der Inhibitoraktivität wurden die Substanzen in DMSO gelöst (Stammlösung c = 1 mg/ml) und in Verdünnung in DMSO 10⁻¹, 10⁻², 10⁻ ³ usw. getestet.

Zur Bestimmung von IC₅₀-Werten wurden die Inhibitor-Stammlösungen in Tris-HCI Puffer, 50 mM, pH 8 weiterverdünnt und in geeigneten Konzentrationen getestet.

Aus der graphischen Darstellung RT-Aktivität gegen den Logarithmus der Konzentration der jeweiligen Testsubstanz wurde die einer 50 %igen Enzymhemmung zugehörige Konzentration ermittelt.

Die Ergebnisse der Untersuchung zeigt Tabelle 2.

**Tabelle 2**

| Verbindung des Beispiels Nr. | Reverse Transkriptase Assay IC₅₀(µg/ml) |
|---|---|
| 2 | 1-10 |
| 8 | 0.1-1 |
| 10 | 0.1-1 |
| 11 | 0.1-1 |
| 20 | 1-10 |
| 37 | 0.1-1 |

Durch die nachfolgenden Beispiele sowie durch den Inhalt der Patentansprüche wird die vorliegende Erfindung näher erläutert.

### Beispiel 2

### (3S)-6-Chlor-3-methyl-3,4-dihydro-1,4,5-triazanaphthalin-2(1H)-on

N-(6-Chlor-3-nitro-2-pyridyl)-alaninmethylester (A) und N-[6-(1-(methoxycarbonyl)ethylamino)-3-nitro-2-pyridyl]-alaninmethylester (B) L-Alaninmethylester Hydrochlorid (8,4 g, 0,06 mol) wurde in 100 ml wasserfreiem N,N-Dimethylformamid gelöst und 16,6 ml (0,12 mol) Triethylamin zugegeben. Anschließend wurden unter kräftigem Rühren 10,7 g (0,05 mol) 2,6-Dichlor-3-nitropyridin in 20 ml wasserfreiem N,N-Dimethylformamid langsam zugetropft, wobei die Reaktionstemperatur auf über 40 °C anstieg. Nach weiteren 3 h bei Raumtemperatur wurde die Reaktionsmischung auf ca. 400 ml Eiswasser gegossen, dreimal mit Essigsäureethylester extrahiert, getrocknet (Natriumsulfat) und eingeengt. Nach Chromatographie an Kieselgel (Essigsäureethylester/Heptan = 1 : 5 dann 1 : 2) isolierte man 9,0 g (69 %) der Verbindung A als gelbes Öl.
¹H-HMR (200 MHz, DMSO-d₆):
- δ =: 1,51 (d, J = 7 Hz, 3 H),
3,69 (s, 3 H),
4,73 (quint., J = 7 Hz, 1 H),
6,89 (d, J = 8.5 Hz, 1 H),
8,48 (d, J = 8.5 Hz, 1 H),
8,67 ppm (d, J = 7 Hz, 1 H).
- MS:: (M + H)⁺ = 260

Eine polarere Fraktion bestand aus 2,4 g (15 %) der Verbindung B als gelber Feststoff vom Schmelzpunkt 113 - 114 °C.
¹H-HMR (200 MHz, DMSO-d₆):
- δ =: 1,35 - 1,45 (m, 6 H),
3,65 (s, 3 H),
3,69 (s, 3 H),
4,46 (quint., J = 7 Hz, 1 H),
4,65 (quint., J = 7 Hz, 1 H),
6,09 (d, J = 9.5 Hz, 1 H),
8,05 (d, J = 9.5 Hz, 1 H),
8,48 (d, J = 7 Hz, 1 H),
8,96 ppm (d, J = 7 Hz, 1 H).
- MS:: (M + H)⁺ = 327

Die Verbindung A (9,0 g, 0,05 mol) wurde in 200 ml Methanol gelöst und unter Raneynickel-Katalyse mit 1 atm Wasserstoff hydriert. Nach beendeter Wasserstoffaufnahme wurde vom Katalysator abgesaugt, und das Lösungsmittel im Vakuum entfernt. Das feste Produkt wurde mit Diethylether angerührt, wobei 2,4 g sauberes Produkt anfielen (Schmelzpunkt 236 - 237 °C).

Der Rückstand aus der Mutterlauge wurde in 150 ml heißem Eisessig gelöst und bei Raumtemperatur stehen gelassen, wobei eine Fällung auftrat. Es wurde eingeengt, mit gesättigter wäßriger Natriumhydrogencarbonatlösung verrührt und abgesaugt, 4,9 g vom Schmelzpunkt 235 - 236 °C. Nach Umkristallisation aus Isopropanol erhielt man 2,4 g des gewünschten Produkts vom Schmelzpunkt 239 - 240 °C.
¹H-NMR (200 MHz, DMSO-d₆):
- δ =: 1,43 (d, J = 7.5 Hz, 3 H),
4,06 (q, J = 7.5 Hz, 1 H),
6,59 (d, J = 7.5 Hz, 1 H),
6,95 (d, J = 7.5 Hz, 1 H),
7,33 (br. s, 1 H),
10,45 ppm (br. s, 1 H).
- MS:: (M + H)⁺ = 198

### Beispiel 5

### (3RS)-4-N-(Isopropenyloxycarbonyl)-3-methyl-3,4-dihydro-1,4,6-triazanaphthalin-2(1H)-on

Aus der Verbindung des Beispiels 1 erhielt man wie für Beispiel 3 beschrieben die gewünschte Verbindung vom Schmelzpunkt 236 °C.
¹H-NMR (200 MHz, DMSO-d₆):
- δ =: 1,20 (d, J = 7 Hz, 3 H),
1,95 (s, 3 H),
4,6 - 5,0 (m, 3 H),
6,97 (d, J = 6 Hz, 1 H),
8,23 (d, J = 6 Hz, 1 H),
8,72 (br. s, 1 H),
11,11 ppm (br. s, 1 H).
- MS:: (M + H)⁺ = 248, (M - (CH₃)₂CO + H)⁺ = 190

### Beispiel 8

### (3S)-6-Chlor-3-methyl-4-N-(3-methyl-2-buten-1-yl)-3,4-dihydro-1,4,5-triazanaphthalin-2(1H)-on

Die Verbindung des Beispiels 2 (988 mg, 5,0 mmol) wurde in 40 ml wasserfreiem 1,2-Dichlorethan suspendiert. Unter Rühren wurden 840 mg (10 mmol) 3,3-Dimethylacrolein und anschließend 1,9 ml (25 mmol) Trifluoressigsäure zugegeben. Man kühlte im Eisbad, trug portionsweise 2,1 g (10 mmol) Natriumtriacetoxyborhydrid ein und ließ 1 h bei 0 °C und weitere 3 h bei Raumtemperatur rühren. Dann wurde das Reaktionsgemisch auf ca. 150 ml gesättigte wäßrige Natriumhydrogencarbonatlösung gegeben, die Phasen getrennt, die wäßrige dreimal mit Dichlormethan nachextrahiert, die vereinigten organischen Extrakte getrocknet (Natriumsulfat) und eingeengt.
Chromatographie an Kieselgel (Essigsäureethylester/Heptan = 1 : 2) lieferte 650 mg (49 %) der gewünschten Verbindung als kristallinen Feststoff vom Schmelzpunkt 136 - 137 °C.
¹H-NMR (200 MHz, DMSO-d₆):
- δ =: 1,24 (d, J = 7 Hz, 3 H),
1,71 (s, 6 H),
3,74 (dd, J = 15, 9 Hz, 1 H),
4,05 (q, J = 7 Hz, 1 H),
4,37 (dd, J = 15, 6 Hz, 1 H),
5,23 (m, 1 H),
6,65 (d, J = 8 Hz, 1 H),
6,98 (d, J = 8 Hz, 1H),
10.61 ppm (s, 1 H).
- MS:: (M + H)⁺ = 266

### Beispiel 9

### (3S)-6-Chlor-1-N-(isopropenyloxycarbonyl)-3-methyl-3,4-dihydro-1,4,5-triazanaphthalin-2(1H)-on

Die Verbindung des Beispiels 2 (988 mg, 5,0 mmol) wurden in 30 ml wasserfreiem Pyridin gelöst. Nach Zugabe von 0,6 ml (5,5 mmol) Chlorameisensäureisopropenylester wurde 4 h bei Raumtemperatur gerührt und anschließend eingeengt. Der Rückstand wurde in Essigsäureethylester gelöst und dreimal mit Wasser gewaschen, getrocknet (Natriumsulfat) und eingeengt. Nach Chromatographie an Kieselgel (Essigsäureethylester/Heptan = 1 : 2) isolierte man die gewünschte Verbindung, die aus Diethylether/Pentan kristallisierte; Ausbeute 380 mg (27 %) vom Schmelzpunkt 86 - 87 °C.
¹H-NMR (200 MHz, DMSO-d₆):
- δ =: 1,35 (d, J = 6 Hz, 3 H),
2,0 (s, 3 H),
4,16 (q, J = 6 Hz, 1 H),
4,95 (s, 2 H),
6,82 (d, J = 9 Hz, 1 H),
7,45 (d, J = 9 Hz, 1 H),
7,60 ppm (br. s, 1 H).
- MS: (M + H)⁺ = 282

### Beispiel 10

### (3S)-6-Chlor-1-N,4-N-bis-(isopropenyloxycarbonyl)-3-methyl-3,4-dihydro-1,4,5-triazanaphthalin-2(1H)-on

Die Verbindung des Beispiels 2 (988 mg, 5,0 mmol) wurden in 80 ml wasserfreiem Dichlormethan gelöst und 593 mg (7,5 mmol) wasserfreies Pyridin zugegeben. Bei 0 °C wurden 663 mg (5,5 mmol) Chlorameisensäureisopropenylester zugetropft. Anschließend ließ man für 3 d bei Raumtemperatur rühren. Es wurde dreimal mit Wasser gewaschen, getrocknet (Natriumsulfat) und eingeengt. Nach Chromatographie an Kieselgel (Aceton/Heptan = 1 : 4) isolierte man 360 mg (25 %) des Produkts als Öl.
¹H-NMR (270 MHz, DMSO-d₆):
- δ =: 1,20 (d, J = 7.5 Hz, 3 H),
1,98 (s, 3 H),
2,01 (s, 3 H),
4,81 (s, 2 H),
4,97 (s, 2 H),
5,04 (q, J = 7.5 Hz, 1 H),
7,49 (d, J = 8 Hz, 1 H),
8,02 ppm (d, J = 8 Hz, 1 H).
- MS: (M + H)⁺ = 366

### Beispiel 11 und Beispiel 12

### (3S)-6-Chlor-3-methyl-4-N-(2-picolyl)-3,4-dihydro-1,4,5-triazanaphthalin-2(1H)-on (Beispiel 11) und (3S)-6-Chlor-1-N-hydroxy-3-methyl-4-N-(2-picolyl)-3,4-dihydro-1,4,5-triazanaphthalin-2(1H)-on (Beispiel 12)

2,6-Dichlor-3-nitropyridin (4,3 g, 0,02 mol) in 50 ml 1,2-Dimethoxyethan und 3,3 ml (0,024 mol) Triethylamin wurden mit 3,9 g (0,02 mol) N-(2-Picolyl)-alaninmethylester 4 h unter Ruckfluß erhitzt. Dann wurde eingeengt, in Essigsäureethylester aufgenommen und zweimal mit Wasser gewaschen. Nach Trocknen (Natriumsulfat) und Einengen blieben 6,9 g N-(6-Chlor-3-nitro-2-pyridyl)-N-(2-picolyl)-alaninmethylester als braunes Öl, das direkt zur Hydrierung eigesetzt wurde. Gelöst in 100 ml Methanol unter Raney Nickel-Katalyse wurde mit 1 atm Wasserstoff hydriert. Nach beendeter Wasserstoffaufnahme wurde vom Katalysator abgesaugt, eingeengt und an Kieselgel chromatographiert (Essigsäureethylester/Methanol /Methanol = 20 : 1). Als unpolarere Fraktion eluierte man 720 mg (12 %) der Verbindung des Beispiels 11 vom Schmelzpunkt 185 °C.
¹H-NMR (200 MHz, DMSO-d₆):
- δ =: 1,27 (d, J = 6.5 Hz, 3 H),
4,11 (q, J = 6.5 Hz, 1 H),
4,43 (d, J = 16 Hz, 1 H),
5,15 (d, J = 16 Hz, 1 H),
6,69 (d, J = 7.5 Hz, 1 H),
7,03 (d, J = 7.5 Hz, 1 H),
7,2 - 7,4 (m, 2 H),
7,75 (dt, J = 8, 2.5 Hz, 1 H),
8,53 (m, 1 H),
10.68 ppm (s, 1 H).
- MS: (M + H)⁺ = 289
Als polarere Fraktion eluierte man 1,75 g (29 %) der Verbindung des Beispiels 12 vom Schmelzpunkt 183 °C.
¹H-NMR (200 MHz, DMSO-d₆):
- δ =: 1,31 (d, J = 7 Hz, 3 H),
4,33 (q, J = 7 Hz, 1 H),
4,45 (d, J = 16 Hz, 1 H),
5,15 (d, J = 16 Hz, 1 H),
6,77 (d, J = 8 Hz, 1 H),
7,2 - 7,4 (m, 2 H),
7,75 (dt, J = 7.5, 2 Hz, 1 H),
8,52 (m, 1 H),
10.92 ppm (s, 1 H).
- MS: (M + H)⁺ = 305
vom Schmelzpunkt 194 - 195 °C.
MS (M + H)⁺ = 208

### Beispiel 17

### (3RS)-3,6-Dimethyl-3,4-dihydro-1,4,5-triazanaphthalin-2(1H)-on

2,3-Diamino-6-methylpyridin (3,0 g, 0,024 mol) und 3,2 ml (0,028 mol) Brenztraubensäureethylester wurden in 100 ml 1,2-Dimethoxyethan 4 h unter Rückfluß erhitzt. Der entstandene Niederschlag (3,9 g), 3,6-Dimethyl-1,4,5-triazanaphthalin-2(1H)-on, wurde abgesaugt, getrocknet und direkt zur Hydrierung eingesetzt. Analog der für Beispiel 1 beschriebenen Reaktion erhielt man 2,27 g (58 %) der gewünschten Verbindung vom Schmelzpunkt 203 - 205 °C
¹H-NMR (200 MHz, DMSO-d₆):
- δ =: 1,28 (d, J = 7 Hz, 3H),
2,20 (s, 3H),
3,94 (dq, J = 7, 2 Hz, 1H),
6,42 (d, J = 9 Hz, 1H),
6,72 (br. s, 1H),
6,84 (d, J = 9 Hz, 1H),
10,20 ppm (br. s, 1H).
- MS: (M + H)⁺ = 178

### Beispiel 18 und Beispiel 19

### (3RS)-4-N-(Isopropenyloxycarbonyl)-3,6-dimethyl-3,4-dihydro-1,4,5-triazanaphthalin-2(1H)-on (Beispiel 18) und (3RS)-1-N,4-N-Bis-(isopropenyloxycarbonyl)-3,6-dimethyl-3,4-dihydro-1,4,5-triazanaphthalin-2(1H)-on (Beispiel 19)

Die Verbindung des Beispiels 17 (0,75 g, 4,2 mmol) wurde in 20 ml wasserfreiem Dichlormethan gelöst und 4 ml wasserfreies Pyridin und 0,72 ml (5,1 mmol) Chlorameisensäureisopropenylester zugegeben. Man ließ 4 h bei 0 °C rühren, wusch mit 1 N wäßriger HCI und gesättigter wäßriger Natriumchloridlösung und trocknete über Natriumsulfat. Nach Abziehen des Lösungsmittels wurde an Kieselgel chromatographiert (Essigsäureethylester/ Heptan = 1 : 2). Als unpolarere Fraktion isolierte man 480 mg (31 %) der Verbindung des Beispiels 19 als Öl.
¹H-NMR (200 MHz, DMSO-d₆):
- δ =: 1,14 (d, J = 7 Hz, 3H),
1,95 (s, 3H),
2,01 (s, 3H),
2,45 (s, 3H),
4,75 - 4,8 (m, 2H),
4,93 (s, 2H),
5,02 (q, J = 7.5 Hz, 1H),
7,22 (d, J = 8 Hz, 1H),
7,81 ppm (d, J = 8 Hz, 1H).
- MS: (M + H)⁺ = 346

Die polarere Fraktion bestand aus 200 mg (18 %) der Verbindung des Beispiels 18 vom Schmelzpunkt 138 - 140 °C.
¹H-NMR (200 MHz, DMSO-d₆):
- δ =: 1,16 (d, J = 7 Hz, 3H),
1,93 (s, 3H),
2,38 (s, 3H),
4,65 - 4,85 (m, 3H),
7,07 (d, J = 8 Hz, 1H),
7,26 (d, J = 8 Hz, 1H),
10.71 ppm (s, 1H).
- MS: (M + H)⁺ = 262

### Beispiel 20

### (3RS)-4-N-(Isopropenyloxycarbonyl)-3,6-dimethyl-3,4-dihydro-1,4,5-triazanaphthalin-2(1H)-thion

Die Verbindung des Beispiels 18 (100 mg, 0,38 mmol) wurde mit 130 mg (0,23 mmol) Lawesson's Reagenz in 10 ml wasserfreiem Toluol 3 h bei 80 °C gerührt. Anschießend wurde eingeengt und an Kieselgel chromatographiert (Essigsäureethylester/Heptan = 1 : 1). Man isolierte 40 mg (38 %) der gewünschten Verbindung vom Schmelzpunkt 120- 121 °C.
¹H-NMR (200 MHz, DMSO-d₆):
- δ =: 1,22 (d, J = 7 Hz, 3H),
1,93 (s, 3H),
2,41 (s, 3H),
4,75 (s, 2H),
5,20 (q, J = 7 Hz, 1H),
7,14 (d, J = 9 Hz, 1H),
7,44 (d, J = 9 Hz, 1H),
12,75 ppm (br. s, 1H).
- MS: (M + H)⁺ = 278

### Beispiel 26

### S-3-Methylthiomethyl-3,4-dihydro-1,4,5-triazanaphthalin-2-(1H)-on

In Analogie zu Beispiel 2, jedoch unter Verwendung nur eines Moläquivalentes Triethylamin, wurden 5 g (27 mmol) (-)-S-Methyl-L-cysteinmethylester mit 3.51 g (22.5 mmol) 2-Chlor-3-nitropyridin umgesetzt (Reaktionsbedingungen: 5 Stunden bei 80 °C). Nach Aufarbeitung wurde an Kieselgel chromatographiert (n-Heptan/Aceton/Methyl-t.-butylether = 5 : 1 : 1). Man erhielt 3.3 g (45 %) N-(3-Nitro-pyrid-2-yl)-(-)-S-methyl-L-cysteinmethylester vom Schmelzpunkt 95 - 97 °C.
Das Produkt wurde direkt weiter umgesetzt.

3.3 g (12.2 mmol) N-(3-Nitro-pyrid-2-yl)-(-)-S-methyl-L-cysteinmethylester wurden analog Beispiel 15 in Methanol unter Raneynickel-Katalyse mit 1 atm Wasserstoff hydriert. Nach Aufarbeitung und Chromatographie an Kieselgel (Ethylacetat/n-Heptan = 2 : 1) erhielt man 891 mg (35 %) S-3-Methylthiomethyl-3,4-dihydro-1,4,5-triazanaphthalin-2-(1H)-on vom Schmelzpunkt 225 - 228 °C.
¹H-NMR (200 MHz, DMSO-d₆): d = 2.07 (s, 3 H), 2.89 (m, 2H), 4.36 (m, 1H), 6.55 (dd, 1H), 6.78 (br s, 1 H), 6.90 (d, 1 H), 7.61 (d, 1H), 10.48 (br s, 1 H) MS: (M + H)⁺ = 210

### Beispiel 32

### 6-Chlor-3-methylthiomethyl-3,4-dihydro-1,4,5-triazanaphthalin-2(1H)-on

### Schmelzpunkt: 202 - 204 °C

¹H-NMR (200 MHz, DMSO-d₆): δ = 2.08 (s, 3 H), 2.73 - 3.05 (m, 2 H), 4.43 (m, 1 H), 6.53 (d, J = 7 Hz, 1 H), 6.91 (d, 1 H), 8.38 (br s, 1 H), 10.62 (br s, 1 H)
MS (M + 1)⁺ = 224

### Beispiel 33

### 4-Isopropoxycarbonyl-3-methylthiomethyl-3,4-dihydro-1,4,5-triazanaphthalin-2(1H)-on

### Schmelzpunkt: 127 - 130 °C

¹H-NMR (200 MHz, DMSO-d₆): δ = 1.22 (d, J = 7 Hz, 3 H), 4.30 (d, J = 7 Hz, 3 H), 2.05 (s, 3 H), 2.49 (dd, J = 9 Zr und 8 Hz, 1 H), 2.73 (dd, J = 5 Hz und 13 Hz, 1 H), 4.90 (m, 1 H), 7.18 (dd, J = 5 Hz und 8 Hz, 1 H), 7.33 (dd, J = 8 Hz und 1 Hz, 1 H), 8.10 (dd, J = 5 Hz und 1 Hz, 1 H), 10.88 (br s, 1 H)
MS (M + 1)⁺ = 296

### Beispiel 36

### 6-Chlor-4-isopropoxycarbonyl-3-methylthiomethyl-3,4-dihydro-1,4,5-triazanaphthalin-2(1 H)-on

### Schmelzpunkt: 198 - 200 °C

¹H-NMR (200 MHz, DMSO-d₆): δ = 1.36 (d, J = 7 Hz, 6 H), 2.08 (s, 3 H), 2.80 - 3.00 (m, 2 H), 4.52 (m, 1 H), 5.14 (hept., J = 7 Hz, 1 H), 6.72 (d, J = 9 Hz, 1 H), 7.21 (d, J = 9 Hz, 1 H), 7.64 (br s, 1 H)
MS (M + 1)⁺ = 330

### Beispiel 37

### 4-lsopropoxycarbonyl-3-methylthiomethyl-3,4-dihydro-1,4,5-triazanaphthalin-2(1H)-thion

### Schmelzpunkt: 188 - 191 °C

¹H-NMR (200 MHz, DMSO-d₆): δ = 1.23 (d, J = 7 Hz, 3 H), 1.28 (d, J = 7 Hz, 3 H), 2.08 (s, 3 H), 2.46 (dd, J = 4 Hz und 14 Hz, 1 H), 2.83 (dd, J = 4 Hz und 14 Hz, 1 H), 4.93 (hept., 1 H), 5.31 (dd, J = 4 Hz und 12 Hz) 7.25 (dd, J = 5 Hz und 8 Hz, 1 H), 8.21 (dd, J = 1 Hz und 5 Hz, 1 H)
MS (M + 1)⁺ = 312

### Beispiel 40

### 3-Methylsulfinylmethyl-4-isopropoxycarbonyl-3,4-dihydro-1,4,5-triazanaphthalin-2(1H)-on

### Schmelzpunkt: 157 - 159 °C

¹H-NMR (200 MHz, DMSO-d₆): δ = 1.22 (d, J = 7 Hz, 3 H), 1.28 (d, J = 7 Hz, 3 H), 2.53 (s, 3 H), 2.78 - 3.13 (m, 2 H), 4.92 (hept., J = 7 Hz, 1 H), 5.20 (m, H), 7.22 (m, 1 H), 7.36 (m, 1 H), 8.13 (m, 1 H), 10.95 (br s, 1 H)
MS (M + 1)⁺ = 312

### Beispiel 41

3-Methylsulfonylmethyl-4-isopropoxycarbonyl-3,4-dihydro-1,4,5-triazanaphthalin-2(1H)-on

### Schmelzpunkt: 209 - 212 °C

¹H-NMR (200 MHz, DMSO-d₆): δ = 1.20 (d, J = 7 Hz, 3 H) 1.28 (d, J = 7 Hz, 3 H), 2.98 (s, 3 H), 3.20 - 3.48 (m, 2 H), 4.92 (hept., 1 H), 5.36 (dd, J = 4 Hz und 9 Hz, 1 H), 7.27 (dd. J = 5 Hz und 8 Hz, 1 H), 7.39 (dd, J = 2 Hz und 8 Hz, 1 H), 7.14 (dd, J = 5 Hz und 2 Hz), 11.03 (br s, 1 H)
MS (M + 1)⁺ = 328

### Beispiel 42

### 4-lsopropoxycarbonyl-3-methylthiomethyl-3,4-dihydro-1,4,5-triazanaphthalin-2(1H)-on

### Schmelzpunkt: 176 °C

¹H-NMR (200 MHz, DMSO-d₆): 1.20 (d, J = 7 Hz, 3 H), 1.26 (d, J = 1 Hz, 3 H), 2.08 (s, 3 H), 4.92 (hept. und m überlagert, 2 H), 7.11 (dd, J = 4 Hz und 8 Hz, 1 H), 7.48 (dd, J = 8 Hz und 1 Hz, 1 H), 7.93 (dd, J = 4 Hz und 1 Hz, 1 H), 9.61 (br s, 1 H), 10.03 (br s, 1 H)
MS (M + 1)⁺ = 311

Außer den oben beschriebenen Beispielen sind die folgenden Verbindungen beispielhaft:
(3S)-6-Chlor-3-methyl-4-N-(3-methyl-2-buten-1-yl)-3,4-dihydro-1,4,5-triazanaphthalin-2(1H)-thion
6-Chlor-3-methyl-4-N-(2-picolyl)-3,4-dihydro-1,4,5-triazanaphthalin-2(1H)-thion
(3RS)-3-Methyl-4-N-(3-methyl-3-buten-1-yl)-3,4-dihydro-1,4,6-triazanaphthalin-2(1H)-thion
6-Chlor-4-N-(isopropenyloxycarbonyl)-3-methylthiomethyl-3,4-dihydro-1,4,5-triazanaphthalin-2(1H)-on
6-Chlor-4-N-(isopropenyloxycarbonyl)-3-methylthiomethyl-3,4-dihydro-1,4,5-triazanaphthalin-2(1H)-thion
6-Chlor-4-N-(isopropyloxycarbonyl)-3-methylthiomethyl-3,4-dihydro-1,4,5-triazanaphthalin-2(1H)-on
6-Chlor-4-N-(isopropyloxycarbonyl)-3-methylthiomethyl-3,4-dihydro-1,4,5-triazanaphthalin-2(1H)-thion

## Patentansprüche

1. Verbindungen der Formel I, 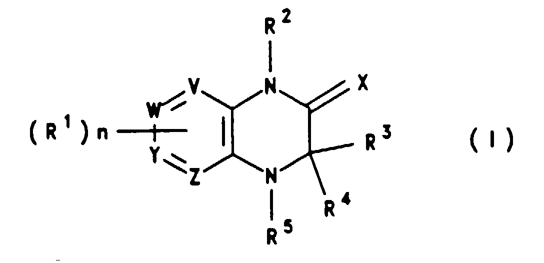 sowie deren tautomere Form der Formel la, 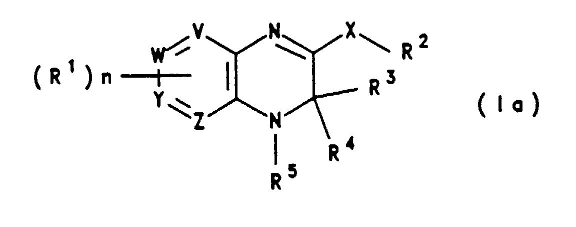 worin bedeuten
n null,
oder eins,
Fluor, Chlor, Trifluormethyl, C₁-C₃-Alkyl, V, W und Y bedeuten CH oder CR¹ und Z bedeutet N,
X bedeutet Sauerstoff oder Schwefel,
R² und R⁵ können gleich oder verschieden, unabhängig voneinander Wasserstoff, Hydroxy,
C₁-C₃-Alkyl, C₂-C₆-Alkenyl, C₁-C₄-Alkyloxycarbonyl, C₂-C₄-Alkenyloxycarbonyl,
oder einen 2-, 3- oder 4-Picolylrest bedeuten,
R³ bedeutet Wasserstoff und R⁴
C₁-C₄-Alkyl, gegebenenfalls substituiert mit C₁-C₂-Alkylthio, C₁-C₂-Alkylsulfonyl oder C₁-C₂-Alkylsulfinyl;
mit Ausnahme der Verbindungen, in denen R² und R⁵ Wasserstoff bedeuten.

2. Verfahren zur Herstellung von Verbindungen der Formel I bzw. la gemäß einem oder mehreren der Ansprüche 1 - 4, dadurch gekennzeichnet, daß
A) zur Herstellung von Verbindungen der Formel I mit X gleich Sauerstoff und den Resten R¹, R², R³, R⁴, R⁵ und n wie in Anspruch 1 definiert, eine Verbindung der Formel II, 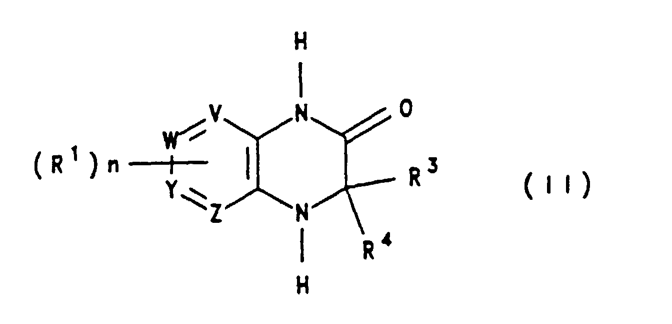 wobei für R¹, R³ und R⁴ die in Anspruch 1 genannten Definitionen gelten, mit einer Verbindung der Formel III,
R-L¹ (III)
wobei R die oben in Anspruch 1 genannten Bedeutungen für R⁵ und R² mit Ausnahme von Wasserstoff, Hydroxy, Acylamino hat und L¹ eine Abgangsgruppe ist, umsetzt
oder daß
B) Verbindungen der Formel I, mit X gleich Schwefel und den Resten R¹, R², R³, R⁴ und R⁵ wie in Anspruch 1 definiert, hergestellt
werden durch Reaktion einer Verbindung der Formel I, wobei X Sauerstoff ist und für R¹ bis R⁵ die in Anspruch 1 beschriebenen Definitionen gelten, mit einem Schwefelungsreagenz,
oder daß
C) Verbindungen der Formel la, wobei X und die Reste R¹ bis R⁵ wie in Anspruch 1 definiert, hergestellt werden, indem man eine Verbindung der Formel IV, 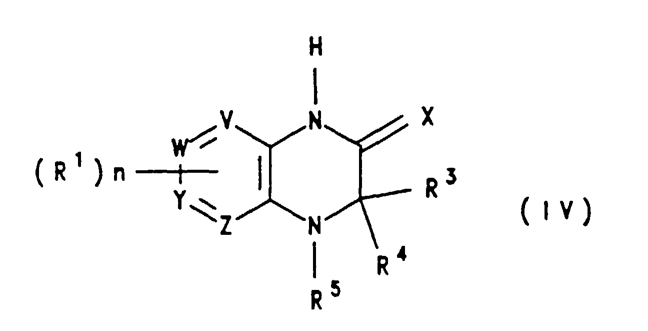 bzw. IVa, 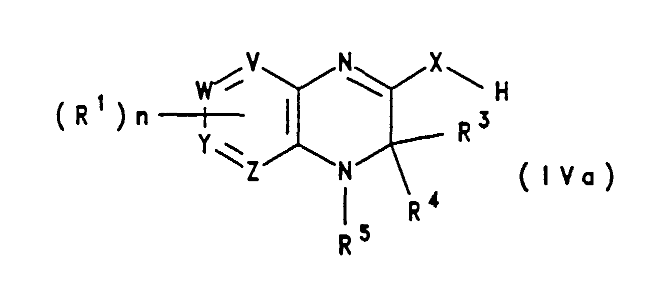 wobei für X, R¹, R³, R⁴ und R⁵ die in Anspruch 1 genannten Definitionen gelten, mit einer Verbindung der Formel III,
R-L¹ (III)
wobei R die oben in Anspruch 1 genannten Bedeutungen für R² mit Ausnahme von Wasserstoff, Hydroxy, hat und L¹ eine Abgangsgruppe ist, umsetzt,
oder daß
D) Verbindungen der Formel I mit X gleich Sauerstoff und den Resten R¹ bis R⁵ wie in Anspruch 1 definiert durch Cyclisierung einer Verbindung der Formel V, 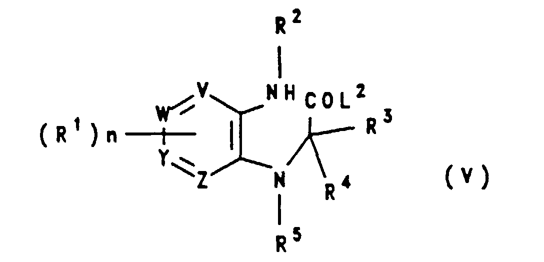 mit R¹ bis R⁵ wie in Anspruch 1 definiert und L² gleich Hydroxy, Alkoxy, ggf. halogeniertes Acyloxy, Chlor, Brom oder Jod, hergestellt werden,
oder daß
F) Verbindungen der Formel I, wobei X gleich Sauerstoff und R¹ bis R⁵ wie in Anspruch 1 definiert sind, hergestellt werden aus Verbindungen der Formel VI, 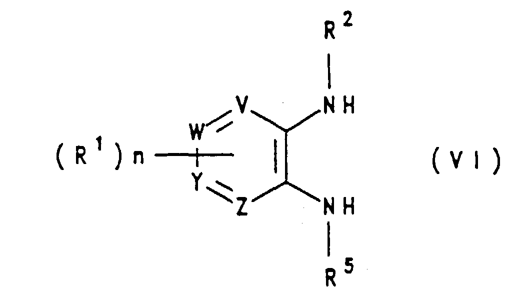 mit R¹, R² und R⁵ wie in Anspruch 1 definiert, durch Umsetzung mit Chloroform oder Bromoform und einer Carbonylverbindung der Formel XIII,
R³-CO-R⁴ (XIII)
mit R³ und R⁴ wie in Anspruch 1 definiert oder mit α-(Trihalogenmethyl)-alkanolen der Formel XIV,
Hal₃C-C(OH)-R³R⁴ (XIV),
worin Hal für Cl, Br oder J steht und in denen R³ und R⁴ wie in Anspruch 1 definiert sind,
oder daß
G) Verbindungen der Formel I, mit X gleich Sauerstoff, R¹, R², R³ und R⁴ wie in Anspruch 1 definiert und R⁵ C₁-C₃-Alkyl; C₂-C₆-Alkenyl;
durch reduktive Alkylierung einer Verbindung der Formel I, wobei R⁵ Wasserstoff und X Sauerstoff sind und für R¹, R², R³ und R⁴ die in Anspruch 1 genannten Definitionen gelten, mit einer Carbonylverbindung der Formel XV,
R"-C(=O)-R"' (XV)
wobei R" und R"' gleich oder verschieden, unabhängig voneinander, C₁-C₃-Alkyl, C₂-C₆-Alkenyl, miteinander verknüpft sein können, hergestellt werden.

3. Verbindungen gemäß Anspruch 1 zur Anwendung als Arzneimittel.

4. Arzneimittel, enthaltend eine wirksame Menge mindestens einer Verbindung der Formel bzw. la gemäß Anspruch 1.

5. Verfahren zur Herstellung eines Arzneimittels gemäß Anspruch 4, dadurch gekennzeichnet, daß die wirksame Menge einer Verbindung der Formel I bzw. la mit üblichen pharmazeutischen Hilfsstoffen in eine geeignete Darreichungsform gebracht wird.

6. Verwendung von Verbindungen gemäß Anspruch 1, wobei die Verbindungen, in denen R² und R⁵ Wasserstoff bedeuten, nicht ausgeschlossen sind, zur Herstellung von Arzneimitteln zur Behandlung von Krankheiten, die durch das HIV verursacht werden.

## Claims

1. A compound of the formula I 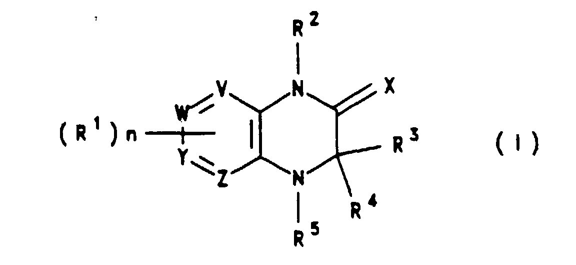 and its tautomeric form of the formula Ia 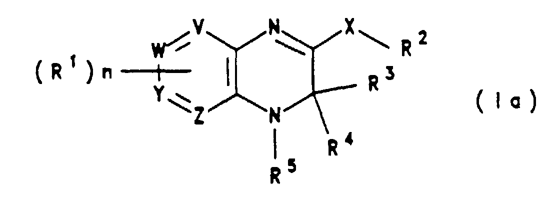 in which
n is zero
or one,
R¹ is
fluorine, chlorine, trifluoromethyl, C₁-C₃-alkyl, V, W and Y are CH or CR¹
X is oxygen or sulfur,
R² and R⁵ can be identical or different and be independently of one another
hydrogen, hydroxyl, C₁-C₃-alkyl, C₂-C₆-alkenyl, C₁-C₄-alkyloxycarbonyl, C₂-C₄-alkenyloxycarbonyl,
or a 2-, 3- or 4-picolyl radical,
R³ is hydrogen and R⁴ is
C₁-C₄-alkyl optionally substituted by C₁-C₂-alkylthio, C₁-C₂-alkylsulfonyl or C₁-C₂-alkylsulfinyl;
with the exception of the compounds in which R² and R⁵ are hydrogen.

2. A process for preparing compounds of the formula I or Ia as claimed in claim 1, which comprises
A) for preparing compounds of the formula I with X equal to oxygen and the radicals R¹, R², R³, R⁴, R⁵ and n as defined in claim 1, reacting a compound of the formula II 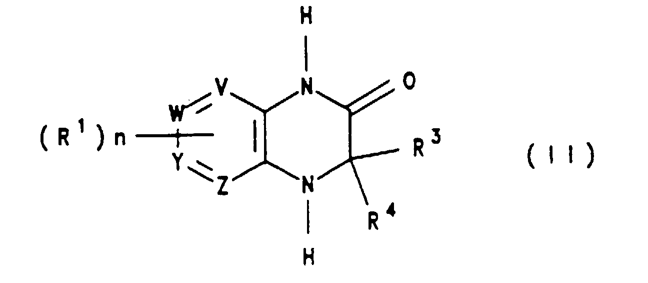 where the definitions mentioned in claim 1 apply to R¹, R³ and R⁴, with a compound of the formula III
R-L¹ (III)
where R has the meanings mentioned above in claim 1 for R⁵ and R² with the exception of hydrogen, hydroxyl, acylamino, and L¹ is a leaving group,
or comprises
B) preparing compounds of the formula I with X equal to sulfur and the radicals R¹, R², R³, R⁴ and R⁵ as defined in claim 1 by reacting a compound of the formula I where X is oxygen, and the definitions described in claim 1 apply to R¹ to R⁵, with a sulfurization reagent,
or comprises
C) preparing compounds of the formula Ia where X and the radicals R¹ to R⁵ are defined as in claim 1 by reacting a compound of the formula IV 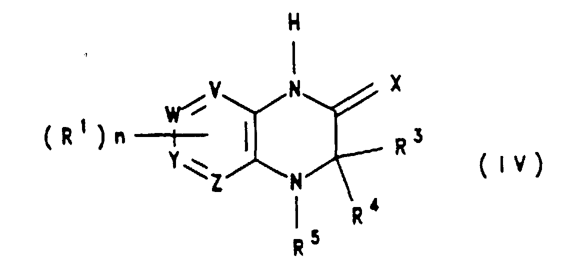 or IVa 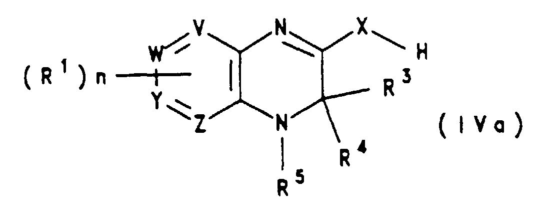 where the definitions mentioned in claim 1 apply to X, R¹, R³, R⁴ and R⁵, with a compound of the formula III
R-L¹ (III)
where R has the meanings mentioned above in claim 1 for R² with the exception of hydrogen, hydroxyl, and L¹ is a leaving group,
or comprises
D) preparing compounds of the formula I with X equal to oxygen and the radicals R¹ to R⁵ as defined in claim 1 by cyclizing a compound of the formula V 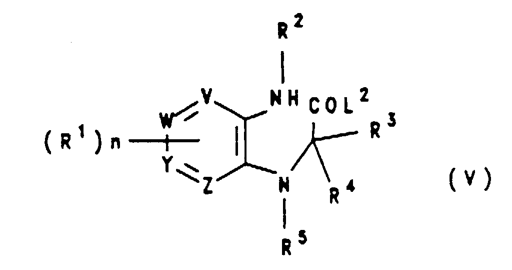 with R¹ to R⁵ as defined in claim 1, and L² equal to hydroxyl, alkoxy, optionally halogenated acyloxy, chlorine, bromine or iodine,
or comprises
F) preparing compounds of the formula I where X equals oxygen and R¹ to R⁵ are defined as in claim 1 from compounds of the formula VI 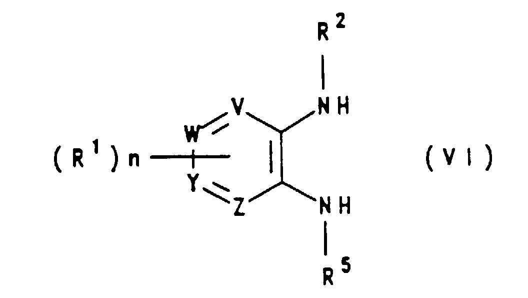 with R¹, R² and R⁵ as defined in claim 1, by reaction with chloroform or bromoform and a carbonyl compound of the formula XIII
R³-CO-R⁴ (XIII)
with R³ and R⁴ as defined in claim 1, or with α-(trihalogenomethyl)alkanols of the formula XIV
Hal₃C-C(OH)-R³R⁴ (XIV)
in which Hal is Cl, Br or I, and in which R³ and R⁴ are defined as in claim 1,
or comprises
G) preparing compounds of the formula I with X equal to oxygen, R¹, R², R³ and R⁴ as defined in claim 1 and R⁵ C₁-C₃-alkyl;
C₂-C₆-alkenyl;
by reductive alkylation of a compound of the formula I where R⁵ is hydrogen and X is oxygen, and the definitions mentioned in claim 1 apply to R¹, R², R³ and R⁴, with a carbonyl compound of the formula XV
R''-C(=O)-R''' (XV)
where R'' and R''' can be identical or different and be independently of one another C₁-C₃-alkyl,
C₂-C₆-alkenyl.

3. A compound as claimed in claim 1 for use as a pharmaceutical.

4. A pharmaceutical containing an effective amount of at least one compound of the formula I or Ia as claimed in claim 1.

5. A process for producing a pharmaceutical as claimed in claim 4, which comprises converting the effective amount of a compound of the formula I or Ia with conventional pharmaceutical ancillary substances into a suitable dosage form.

6. The use of compounds as claimed in claim 1, the compounds in which R² and R⁵ are hydrogen not being excluded, for the production of pharmaceuticals for the treatment of diseases which are caused by HIV.

## Revendications

1. Composés de formule I : 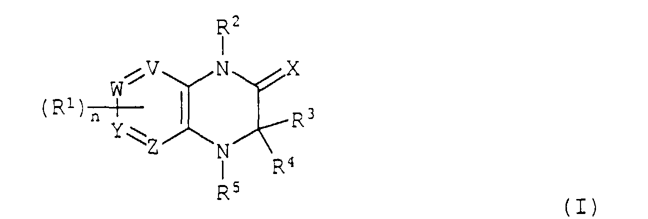 ainsi que leurs formes tautomères de formule la : 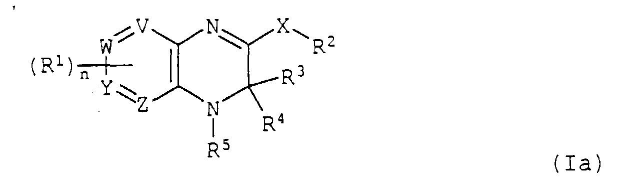 formules dans lesquelles :
n vaut zéro ou un ;
R¹ est un fluor, chlore, trifluorométhyle ou alkyle en C₁ à C₃;
V, W et Y sont CH ou CR¹ ;
Z est N ;
X est un oxygène ou un soufre ;
R² et R⁵ peuvent être identiques ou différents et sont indépendamment l'un de l'autre un hydrogène, hydroxy, alkyle en C₁ à C₃, alcényle en C₂ à C₆, alkyloxycarbonyle en C₁ à C₄, alkylénoxycarbonyle en C₂ à C₄, ou bien un résidu 2-, 3- ou 4-picolyle ;
R³ est un hydrogène ; et
R⁴ est un alkyle en C₁ à C₄ le cas échéant substitué par un alkylthio en C₁-C₂, alkylsulfonyle en C₁-C₂ ou alkylsulfinyle en C₁-C₂,
à l'exception des composés dans lesquels R² et R⁵ sont des hydrogènes.

2. Procédé de préparation de composés de formule I ou la selon l'une ou plusieurs des revendications 1 à 4, caractérisé en ce que
A) pour produire des composés de formule I dans laquelle X est un oxygène et les résidus R¹, R², R³, R⁴ et R⁵ ainsi que n ont les significations indiquées en 1), on fait réagir un composé de formule II : 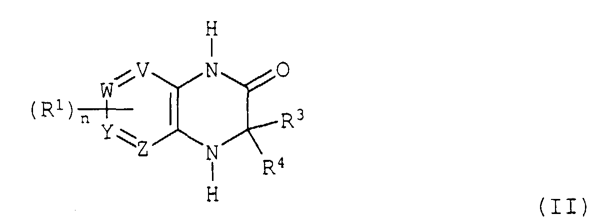 dans laquelle R¹, R³ et R⁴ ont les significations indiquées en 1), avec un composé de formule III :
R-L¹ (III)
dans laquelle R a les significations indiquées en 1) pour R⁵ et R² sauf hydrogène, hydroxy et acylamino, et L¹ est un groupe partant ;
ou bien
B) on prépare des composés de formule I, dans laquelle X est un atome de soufre et les résidus R¹, R², R³, R⁴ et R⁵ ont les significations indiquées en 1), en faisant réagir un composé de la formule I, dans laquelle X est un atome d'oxygène et les résidus R¹ à R⁵ ont les significations indiquées en 1), avec un réactif de sulfuration ;
ou bien
C) on prépare des composés de formule la, dans laquelle X et les résidus R¹ à R⁵ ont les significations indiquées en 1), en faisant réagir un composé de formule IV : 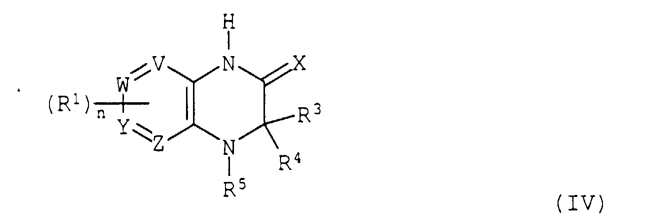 ou IVa 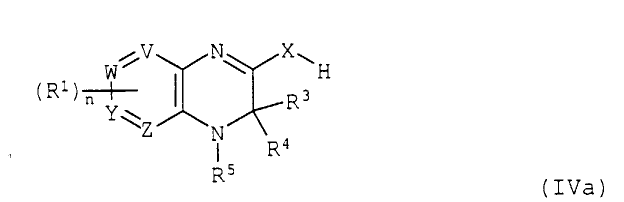 dans lesquelles X, R¹, R³, R⁴ et R⁵ ont les significations indiquées en 1), avec un composé de formule III :
R-L¹ (III)
dans laquelle R a les significations indiquées en 1) pour R² sauf hydrogène et hydroxy, et L¹ est un groupe partant ;
ou bien
D) on prépare des composés de formule I, dans laquelle X est un oxygène et les résidus R¹ à R⁵ ont les significations indiquées en 1), par cyclisation d'un composé de formule V : 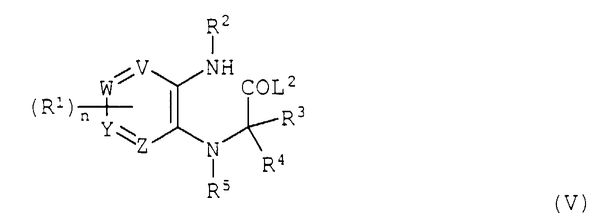 dans laquelle R¹ à R⁵ ont les significations indiquées en 1) et L² est un hydroxy, alcoxy, acyloxy le cas échéant halogéné, chlore, brome ou iode ;
ou bien
F) on prépare des composés de formule I, dans laquelle X est un oxygène et R¹ à R⁵ ont les significations indiquées en 1), à partir de composés de formule VI : 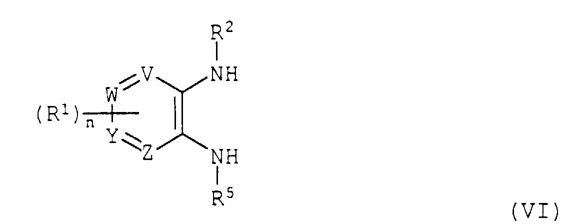 dans laquelle R¹, R² et R⁵ ont les significations indiquées en 1), par réaction avec du chloroforme ou bromoforme et un composé carbonyle de formule XIII :
R³-CO-R⁴ (XIII)
dans laquelle R³ et R⁴ ont les significations indiquées en 1), ou avec des α-(trihalogénométhyl)-alcanols de formule XIV :
Hal₃C-C-OH)-R³R⁴ (XIV)
dans laquelle Hal est Cl, Br ou I et R³ et R⁴ ont les significations indiquées en 1) ;
ou bien
G) on prépare des composés de formule I, dans laquelle X est un oxygène, R¹, R², R³ et R⁴ ont les significations indiquées en 1) et R⁵ est un alkyle en C₁ à C3 ou un alcényle en C₂ à C₆, par alkylation réductrice d'un composé de formule I, dans laquelle R⁵ est un hydrogène, X un oxygène, et R¹, R², R³ et R⁴ ont les significations indiquées en 1), avec un composé carbonyle de formule XV :
R"-C(=O)-R"' (XV)
dans laquelle R" et R"' sont identiques ou différents et représentent indépendamment l'un de l'autre un alkyle en C₁ à C₃ ou un alcényle en C₂ à C₆.

3. Composés selon la revendication 1 pour une utilisation comme médicaments.

4. Médicament contenant une quantité efficace d'au moins un composé de formule I ou la selon la revendication 1.

5. Procédé de production d'un médicament selon la revendication 4, caractérisé en ce que la quantité efficace d'un composé de formule I ou la est amenée dans une présentation appropriée avec des additifs et adjuvants pharmaceutiques usuels.

6. Utilisation de composés selon la revendication 1, dans laquelle les composés dans lesquels R² et R⁵ signifient l'hydrogène ne sont pas exclus, pour la fabrication de médicaments destinés au traitement de maladies provoquées par le VIH.
